# EUROPEAN PATENT SPECIFICATION

(11) **EP 4 021 349 B1**
(45) Date of publication and mention of the grant of the patent: **22.01.2025**
(21) Application number: 20768463.0
(22) Date of filing: 27.08.2020
(51) Int. Cl.: A61F 2/24

(54) **DEVICE FOR MITRAL REPAIR INCLUDING PAPILLARY MUSCLE RELOCATION**
VORRICHTUNG ZUR MITRALREPARATUR EINSCHLIESSLICH PAPILLARMUSKULATUR
DISPOSITIF DE RÉPARATION DE LA VALVULE MITRALE COMPRENANT LE REPOSITIONNEMENT DES MUSCLES PAPILLAIRES

(30) Priority: 28.08.2019 US 201962892708 P
(43) Date of publication of application: 06.07.2022
(73) Proprietor: Boston Scientific Scimed Inc., Maple Grove, Minnesota 55311 (US)
(72) Inventor: CLARK, Bryan A., Forest Lake, Minnesota 55025 (US); FLANAGAN, Aiden, Galway (IE); O'SULLIVAN, Sean, Galway (IE); HORGAN, Fergal, Shrule Co. Mayo (IE)
(74) Representative: Peterreins Schley
(86) International application number: PCT/US2020/048155
(87) International publication number: WO 2021/041639

(56) References cited:
- WO-A1-2018/126188
- WO-A2-2011/034973
- US-A1- 2007 016 287

## Description

### FIELD

The present disclosure relates generally to the field of implantable medical devices and more particularly to implantable devices, systems, and methods for adjusting heart features.

### BACKGROUND

Mitral insufficiency (MI) is a form of heart disease where the mitral annulus excessively dilates and the valve leaflets no longer effectively coapt during systolic contraction. Regurgitation occurs during ventricular contraction and cardiac output decreases.

An annuloplasty procedure may be performed to restore the physiological form and function of the mitral annulus. Annuloplasty procedures may involve surgically implanting a ring around the mitral annulus to restore a diameter of the patient's mitral annulus to that of a healthy state where the valve leaflets properly coapt and mitral regurgitate flow is minimized. Additionally, sub-valvular repair procedures such as repositioning of papillary muscles or repairing chordae within the left ventricle may be performed.

Due to the invasive nature of the surgical approaches to mitral valve repair, several transcatheter techniques have been developed to emulate surgical approaches. Delivery catheters may extend up to 52" in length and may carry mitral valve or sub-valvular repair components for distal delivery to a treatment site. The challenges of advancing components through a catheter include difficulties maintaining an orientation, spacing and/or integrity of the components, as well as component entanglement.

US 2007/0016287 A1 discloses an implantable device for controlling the shape and/or size of an anatomical structure or lumen. WO 2011/034973 A2 discloses a device for the endovascular repair of cardiac valves. WO 2018/126188 A1 discloses devices for transvascular prosthetic chordae tendinea implantation.

### SUMMARY

The present invention is directed to a delivery catheter for cardiac repair components as set forth in the appended claims. According to the invention, the delivery catheter includes a first channel extending from a proximal end to a distal end of the delivery catheter, the first channel configured for axial translation of a first component to a treatment site. The delivery catheter further includes a second channel, extending from the proximal end of the delivery catheter to the distal end of the delivery catheter, the second channel configured for axial translation of a second component to the treatment site, where the first component is coupled to the second component, and the first component is configured for axial translation through the first channel in coordination with axial translation of the second component through the second channel for concurrent deployment of the first component and second component from the delivery catheter. An axially translatable opening is provided between the first channel and the second channel. The delivery catheter further includes a guide catheter disposed within the delivery catheter, where the first channel is disposed between the guide catheter and the delivery catheter. The delivery catheter includes an anchor catheter disposed within the guide catheter, the anchor catheter including a slit extending through the anchor catheter from a distal end of the anchor catheter to a proximal end of the anchor catheter, the anchor catheter providing the second channel. The guide catheter includes a window extending therethrough, the window slidably disposed over the slit of the anchor catheter, where the window of the guide catheter and slit of the anchor catheter cooperate to provide the axially translatable opening between the first channel and the second channel.

According to various embodiments, the first channel and second channel may be configured to couple the first component to the second component while limiting interaction between the first component and the second component. The second channel may be configured to limit rotational movement of the second component during translation of the second component through the second channel, where the first channel is disposed within a portion of the second channel defined by the second component.

In one embodiment, the second channel may include a bore sized to limit rotation of the second component, and the first channel may be disposed between features of the second component. In one embodiment, the bore may correspond in size and shape to the second component. The first component may include a suture and the second component may include an anchor having a suture coupling, and the suture may extend through the suture coupling of the anchor. In one embodiment, the suture coupling may be disposed within the second channel. In other embodiments, the suture coupling may be disposed within the first channel.

In one embodiment, the delivery catheter may include a plurality of anchors, and the suture may extend from a proximal handle of the delivery catheter through the plurality of anchors and back to the proximal handle. In other embodiments, the delivery catheter may include a plurality of anchors, and the suture may be fixedly attached to a first anchor of the plurality of anchors and extend through each of the plurality of anchors to the proximal end of the delivery catheter.

In one embodiment, the second component may include a body and a coupler for engaging the first component, the first channel may be configured to carry the coupler and the second channel may be configured to carry the body.

According to another aspect, a cardiac repair system includes a suture, a plurality of anchors, and a delivery catheter. The delivery catheter includes a first channel extending from a proximal end to a distal end of the delivery catheter, the first channel configured for axial translation of the suture to a treatment site, a second channel, extending from the proximal end of the delivery catheter to the distal end of the delivery catheter, the second channel configured for axial translation of the plurality of anchors to the treatment site. A portion of the suture may be coupled to an anchor and the delivery catheter may be configured for axial translation of the portion of the suture through the first channel in coordination with axial translation of the anchor through the second channel for concurrent deployment of the portion of the suture and the anchor without interference.

In some embodiments, the second channel may include a bore sized to limit rotation of at least one anchor of the plurality of anchors, and the first channel may be defined by features of the at least one anchor. The bore may correspond in size and shape to the at least one anchor. The cardiac repair system may further include an axially translatable opening disposed between the first channel and the second channel.

In some embodiments, the cardiac repair system may include a guide catheter disposed within the delivery catheter, the first channel disposed between the guide catheter and the delivery catheter. An anchor catheter may be disposed within the guide catheter, the anchor catheter including a slit extending through the anchor catheter from a distal end of the anchor catheter to a proximal end of the anchor catheter, the anchor catheter providing the second channel. The guide catheter may include a window extending therethrough, the window slidably disposed over the slit of the anchor catheter, where the window of the guide catheter and slit of the anchor catheter cooperate to provide the axially translatable opening between the first channel and the second channel

The present disclosure also provides a method for cardiac repair includes the steps of: coupling a first anchor to a suture, axially translating the suture through a first channel of a delivery catheter, axially translating the first anchor through a second channel of the delivery catheter in coordination with axial translation of the suture, concurrently deploying a first portion of the suture and the first anchor to a treatment site, axially translating a second anchor through a second channel of the delivery catheter in coordination with axial translation of the suture, and concurrently deploying a second portion of the suture and the second anchor to a treatment site.

In some embodiments, the delivery catheter includes an axially translatable opening disposed between the first channel and the second channel, and the step of axially translating the first anchor through a second channel of the delivery catheter in coordination with axial translation of the suture may include the steps of: introducing either a coupling portion of the first anchor into the first channel or introducing the first portion of the suture into the second channel to form a join between the first anchor and the first portion of the suture, aligning the axially translatable opening between the first channel and the second channel with the join between the first anchor and the first portion of the suture and advancing the axially translatable opening of the delivery catheter in coordination with the join to minimize an exposure between the first channel and the second channel.

### BRIEF DESCRIPTION OF THE DRAWINGS

Non-limiting embodiments of the present disclosure are described by way of example with reference to the accompanying figures, which are schematic and not intended to be drawn to scale. In the figures, each identical or nearly identical illustrated component is typically represented by a single numeral. For purposes of clarity, not every component is labeled in every figure, nor is every component of each embodiment shown where illustration is not necessary to allow those of ordinary skill in the art to understand the disclosure. In the figures:
FIG. 1 is a diagram of a portion of a heart in which delivery catheters such as those disclosed in various embodiments herein may be deployed;
FIGS. 2A and 2B are perspective views of one embodiment of a delivery catheter disclosed herein.
FIGS. 3A and 3B illustrate one embodiment of an anti-tangle device that maybe be used with delivery catheters such as those disclosed herein;
FIG. 4 illustrates a method of delivering repair components to a heart chamber according to an embodiment disclosed herein;
FIGS. 5A-5C illustrate another method of delivering repair components to a heart chamber according to an embodiment disclosed herein.
FIGS. 6A - 6D illustrate various views of embodiments of a delivery catheter system configured to maintain one of separation, spacing and/or orientation between repair components during translation of components to a treatment site;
FIG. 7 is a view of one embodiment of a delivery catheter configured to maintain one of separation, spacing and/or orientation between repair components during translation of components to a treatment site;
FIG. 8 is a view of one embodiment of a delivery catheter configured to maintain one of separation, spacing and/or orientation between repair components during translation of components to a treatment site;
FIGS. 9A-9C provide various views of one embodiment of a delivery catheter and repair components configured to maintain one of a separation, spacing and/or orientation between repair components during translation of components to a treatment site; and
FIG. 10 is a diagram of a heart used to describe delivery of cardiac repair components according to embodiments of methods disclosed herein.

### DETAILED DESCRIPTION

The present invention is directed to a delivery catheter for cardiac repair components as set forth in the appended claims. Where in the following the unit "inch" is employed, this conversion applies: 1 inch = 25.4 mm

A delivery catheter as disclosed herein may be configured in various embodiments to maintain an orientation, a spacing and/or separation of annuloplasty or other cardiac repair components as they are translated through the delivery catheter to a treatment site. For example, cardiac repair components may include one or more anchors coupled by sutures. The anchors may be deployed to a heart wall, annulus or other heart feature and the suture cinched or otherwise tightened to adjust the spacing between the deployed anchors. Such a system may be used to bring tissue features of the heart closer together, for example, for valvular and/or sub-valvular repair procedures such as annuloplasty and repair, replacement and/or repositioning of a valve leaflet, a papillary muscle or chordae to improve valve function.

According to one aspect, the delivery catheter may be configured in various embodiments to minimize the potential for entanglement between repair components such as between sutures and coupled anchors. The embodiments include embodiments that separate an anchor translation channel from a suture translation channel while maintaining the coupling between the anchor and the suture. In some embodiments, the coupling may include a suture coupling of an anchor, the suture coupling comprising a suture lumen extending through a portion of the anchor and configured to attach to or to slideably carry the suture. In various embodiments, the suture coupling is isolated from other repair components. In some embodiments wherein the anchor comprises talons, barbs or the like on a distal end, the delivery catheter is configured such that the suture coupling is spaced apart from the talons to minimize the potential for entanglement. In some embodiments, the suture coupling may be disposed in a channel that is the same as or separate from the anchor talons. In some embodiments, the delivery catheter may be configured to maintain an anchor orientation during translation, which may further reduce the potential of entanglement of components during delivery.

These and other beneficial aspects of a delivery catheter configured to maintain one of separation, spacing and/or orientation of anchors during annuloplasty or are described in more detail below. It should be noted that, although embodiments of the present disclosure may be described with specific reference to papillary muscles, the principles disclosed herein may be readily adapted to facilitate reconstruction of various heart features, including but not limited to a mitral or tricuspid valve annulus and/or may similarly benefit any other dilatation, valve incompetency, valve leakage and other similar heart failure conditions.

As used herein, the term "distal" refers to the end farthest away from the medical professional when introducing a medical device into a patient, while the term "proximal" refers to the end closest to the medical professional when introducing a medical device into a patient.

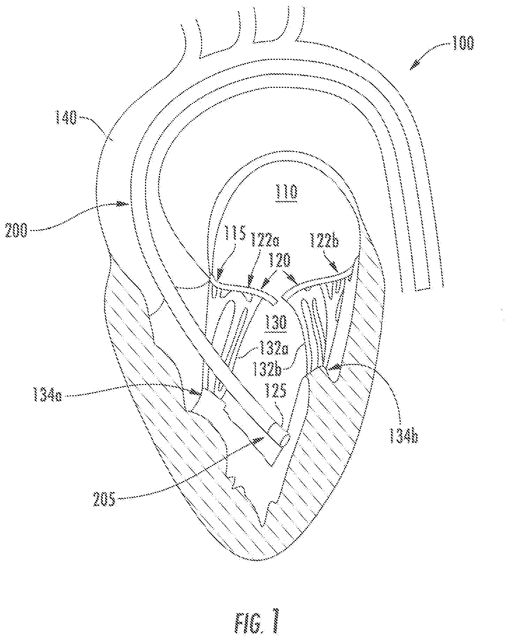

FIG. 1 is a diagram of a left chamber of a heart 100, including a left atrium 110 separated from a left ventricle 130 by mitral valve 120. The mitral valve 120 includes an anterior leaflet 122a and a posterior leaflet 122b which are attached in a healthy heart to respective papillary muscles 134a, 134b via chordae tendineae 132a, 132b. The papillary muscles 134a, 134b contract to prevent inversion or prolapse of the valves 122a, 122b on contraction of the left ventricle 130. A mitral annulus 115 comprises a fibrous ring that, in a healthy heart is saddle shaped and of a diameter to enable the valves to close, or coapt, during systolic contraction.

In a diseased heart, one or more of the chordae tendineae 132a, 132b may be stretched or ruptured, resulting in a flailing leaflet 122a, 122b that no longer effectively closes, resulting in regurgitation. Alternatively, or in conjunction, the mitral annulus 115 may become stretched or deformed, and the valves may also fail to close as a result.

To repair the heart failure condition, repair components may be transluminally deployed to the heart 100. In FIG. 1, a delivery catheter 200 is shown advanced through a femoral artery to the aorta 140 and into the left ventricle 130 for transfemoral retrograde delivery of repair components. Depending upon the heart feature that is to be repaired it is appreciated that the delivery catheter is not limited by the manner in which it is introduced to the heart 100. For example, to deliver repair components to a left atrium, a transapical or transseptal delivery pathway may be used with embodiments of the delivery catheter and system disclosed herein.

In one embodiment, the delivery catheter 200 may have a steerable distal end 205 to facilitate navigation of repair components 125 that are disposed within the distal end of the delivery catheter 200 into a heart chamber. During delivery, a distal guidewire (not shown) may assist with transluminal navigation. Upon arrival at the treatment site, repair components 125 may be advanced through the distal end 205 of the delivery catheter 200 as part of the cardiac procedure.

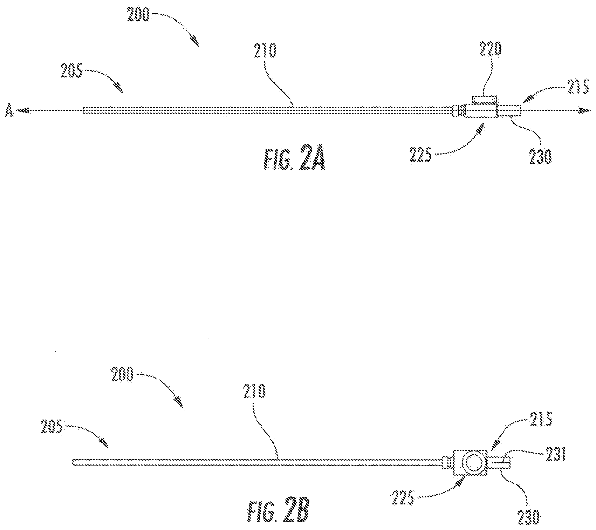

One embodiment of a delivery catheter 200 in accordance with the invention is shown from a side perspective in FIG. 2A and a top-down perspective in FIG. 2B. The delivery catheter 200 is shown to include a shaft 210 having a steerable distal end 205 aligned along an axis A to a handle 225 of a proximal end 215. The steerable shaft 210 may include embedded pull cables coupled to mechanisms in the handle 225 and configured to deflect the distal end 205 of the shaft 210 as it travels through the lumens of arteries or veins to a treatment site such as a cardiac cavity. In one embodiment, the delivery catheter 200 may comprise one or more sheaths, each sheath comprising a composite of layers of thermoplastic elastomer (TPE), for example PEBAX provided by ARKEMA corporation of Colombes France. Alternatively, nylon, polyurethanes, polyester, silicone or other similar materials may be used to provide thin walls that may be extruded and layered over braided wires or coils for tensile and hoop strength, although the disclosed system is not limited to any particular material composition for the delivery catheter. In some embodiments, the length of the shaft 210 may range from between 24"-52", and more particularly between 42"-46". In one embodiment, the inner diameter may range between 24-31 Fr, and the outer diameter may range between 26 Fr and 34 Fr or more. In an exemplary embodiment, an inner diameter may be, for example 28 Fr and the outer diameter may be 32 Fr.

According to one aspect, the delivery catheter 200 includes a delivery catheter lumen extending axially through catheter 200 from the proximal end 215 to the distal end 205 as shown by line 'A' of FIG. 2A. As described in more detail below, in various embodiments the anchor lumen may be apportioned into one or more channels separating translation of the anchors, sutures and/or anchor/suture coupling from other repair components during deployment.

The handle 225 may include a steering control mechanism such as dial 220 which may control the deflection of the distal end 205 of the delivery catheter 200 during transluminal navigation. Alternative steering control mechanisms may include, for example, thumbwheels, dials, knobs, switches and the like.

**In** some embodiments, the handle 225 may further include an anti-tangle device configured to align sutures to reduce tangling that would frustrate an anchoring procedure, for example an anchor feed cylinder 230.

FIG. 2B is a top down perspective view of the delivery catheter 200, illustrating the shaft 210, coupled to its proximal end to the handle 225 which in turn is coupled to the anchor feed cylinder 230. **In** FIG. 2B, anchor feed cylinder 230 is shown to include a slot 231 that extends through its proximal end and may be configured to separate translation of sutures and anchors through the delivery catheter 200. For example, the anchor feed cylinder 230 may be configured to align an anchor with a first channel extending from the proximal end 215 to /through the distal end 205, and to align the suture with a second channel that extends from the proximal end 215 to/through the distal end 205 to enable translation of a coupled suture/anchor pair through the shaft without entanglement.

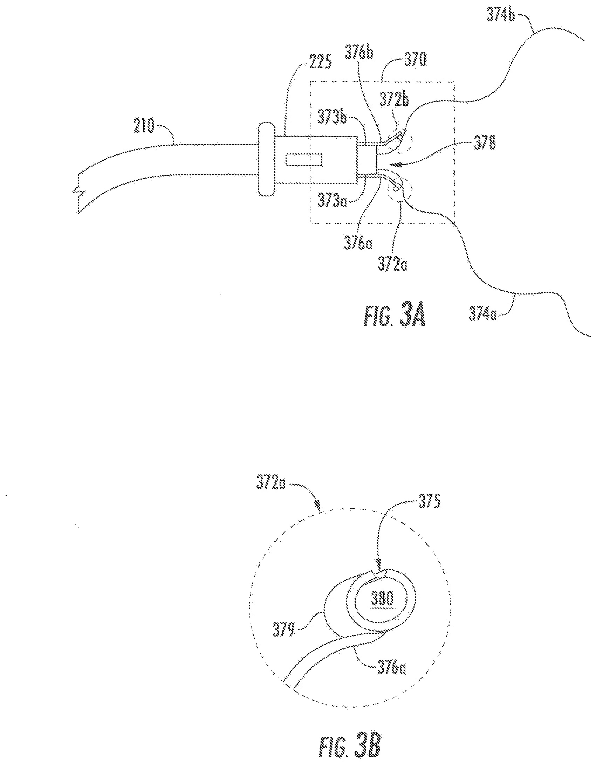

FIGS. 3A and 3B illustrate one embodiment of an anti-tangle device 370 that may be used to reduce suture interference during a suturing procedure. FIG. 3A illustrates a handle 225 configured to control a delivery sheath 210 as described above. The anti-tangle device 370 may be disposed at the proximal end of the handle 225. **In** various embodiments, the anti-tangle device 370 may be integral with or detachable from the proximal end of the handle 225. The anti-tangle device 370 is shown to include a body 378 coupled to or integral with the proximal end of the handle. **In** one embodiment, the body 378 may comprise a ring or similar structure having one or more openings extending therethrough to enable sutures and anchors to be fed through the handle 225 for deployment to a treatment site. A pair of arms 376a, 376b include respective distal ends 373a, 373b fixedly or releasably attached to spaced apart locations upon or within the body 378 of the anti-tangle device 370. **In** the illustrated embodiment, arm 376a is shown disposed on an opposing side of body 378 from arm 376b, although the present disclosure is not so limited.

The arms 376a, 376b further include proximal ends supporting alignment heads 372a, 372b. The arms 376a, 376b are advantageously configured to displace the alignment heads 372a, 372b from each other. Displacing the alignment heads 372a, 372b allows sutures 374a, 374b to be fed through the alignment heads and into the handle 225 without interference and associated entanglement issues. It should be noted that although two arms are shown, similar solutions which have a single arm comprising spaced apart branches, each branch supporting one or more of the alignment heads are within the scope of this disclosure.

FIG. 3B is a close up perspective view of one embodiment of alignment head 372a. The alignment head 372a is shown to include an eyelet 379 disposed on the proximal end of arm 376a, the eyelet 379 having a bore 380 extending therethrough and sized in diameter to slideably support a suture line. A slot 375, extending through the eyelet 379 into the bore 380, enables the suture to be introduced into the bore 380, to allow the bore 380 to guide translation of the suture into the handle 225 (FIG. 3A) to reduce opportunity for entanglement that may frustrate a cardiac procedure.

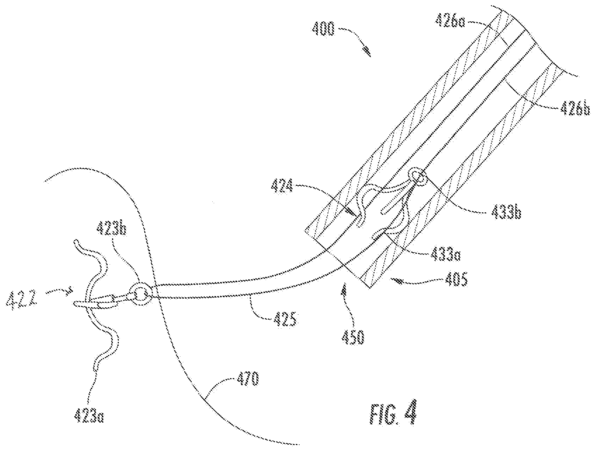

FIG. 4 illustrates a distal end 405 of a delivery catheter 400 having an anchor lumen 450 extending therethrough. The delivery catheter 400 is shown deploying repair components including anchors 422 and 424 and a suture 425. The anchors 422 and 424 may be deployed for anchoring in a heart feature 470 to repair heart function. Anchor 422 is shown to include a talon 423a disposed at a distal end, and a suture coupler including an eyelet 423b disposed at the proximal end. Anchor 424 similarly comprises a talon 433a disposed at a distal end and an eyelet 433b disposed at a proximal end. Although the talon 423a and eyelet 423b combination are shown, the present disclosure is not limited to any particular anchor or suture coupling mechanism. Rather, anchors comprising barbs, hooks, screws, helical anchors and the like may be readily substituted herein depending upon the form of repair. In addition, other suture coupling mechanisms enabling fixed or slidable translation of the suture relative to a proximate anchor, including clips or lumens extending through or partially through an anchor body may alternatively be used.

According to one aspect, the anchors 422, 424 may be comprised of a shape memory material, for example a copper-aluminum-nickel, a nickel-titanium (NiTi) alloy or other alloy of zinc, copper, gold and/or iron. In one embodiment, the anchors 422, 424 may include a first configuration, such as the configuration of anchor 424, that facilitates translation of the anchor 424 through the anchor lumen 450, and may revert to a second configuration, such as the configuration of anchor 422, when released from the distal end 405 of the delivery catheter 400 to a treatment site, the second configuration exposing the talons 423a to tissue.

In one embodiment, the suture 425 may be a continuous line having a first end 426a that loops around an initially deployed anchor, such as anchor 422, and returns back through the anchor lumen 450 to the proximal end the delivery catheter 400 at a second end 426b. Subsequent anchors, such as anchor 424, may then be translated over either the first end 426a or the second end 426b of the suture line 425 to a desired treatment location.

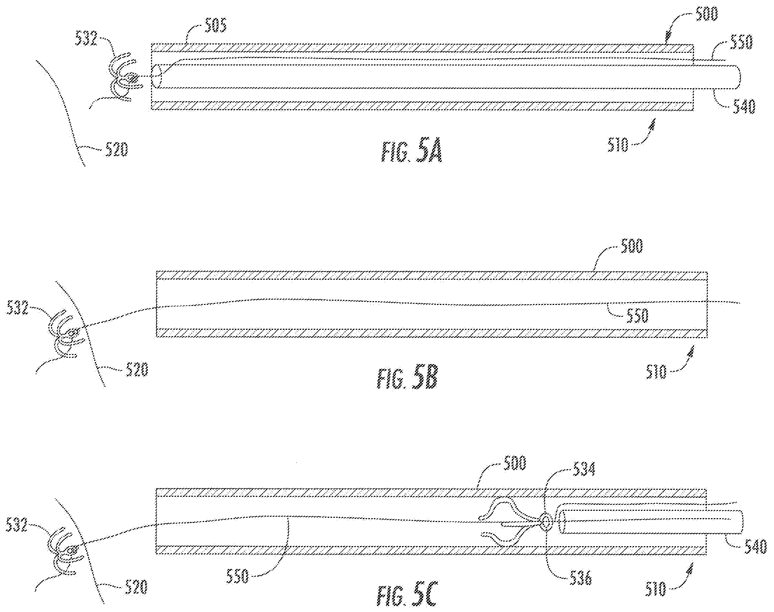

In other embodiments, the suture may be terminated at, or otherwise affixed to, the first, distal most anchor and fed through subsequently translated anchors, for example as shown in FIGS. 5A - 5C. FIG 5A illustrates a delivery catheter 500 having an anchor 532 deployed through a distal end 505 of the delivery catheter using a push rod 540. Delivery catheter 500 may include a handle (not shown) coupled to its proximal end 510. **In** the embodiment of FIG. 5A, the suture 550 is shown affixed to the initial anchor 532, and the push rod 540 is shown advancing the anchor 532 towards a heart feature 520.

**In** FIG. 5B, the anchor 532 has been affixed to the heart feature 520, and the push rod withdrawn from the delivery catheter 500. The suture 550 extends from the affixed anchor 532 through the proximal end 510 of the delivery catheter 500.

In FIG. 5C, a second anchor 534 is advanced through the delivery catheter 500. In one embodiment, the anchor 534 may be threaded over the suture 550, for example through eyelet 536 of anchor 534. The anchor 534 may be advanced from the proximal end 510 of the delivery catheter 500 over suture 550 using push rod 540 and directed towards the heart feature 520, for placement with anchor 532, or may be directed towards another heart feature.

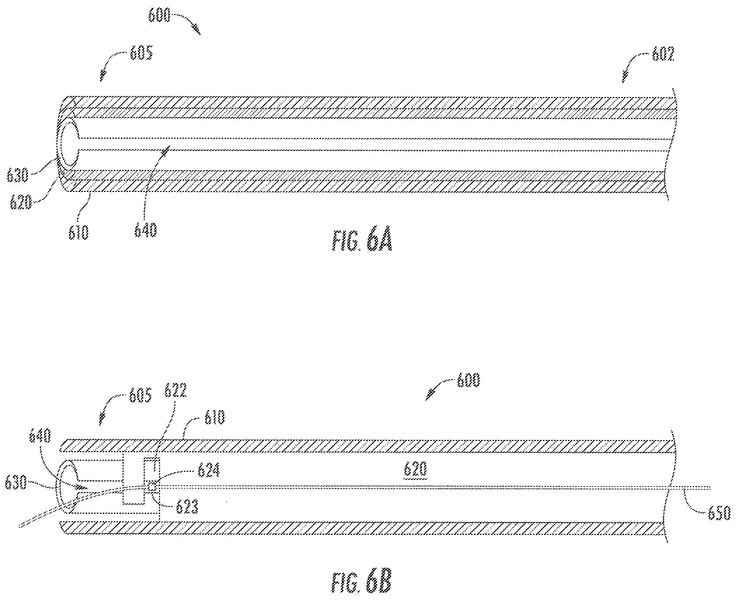

In accordance with the invention, FIGS. 6A-6D illustrate perspective and cross-sectional views of a distal end of one embodiment of a delivery catheter 600 configured to maintain one or more of a separation, orientation and/or spacing between repair components. The delivery catheter 600 is shown in FIG. 6A to be comprised of a plurality of sheaths, including a delivery catheter sheath 610, a guide sheath 620 and an anchor sheath 630, wherein the anchor sheath 630 is translatably disposed within the guide sheath 620, and the guide sheath is translatably disposed within the delivery catheter sheath 610. According to one aspect, replacement components such as anchors may be translated through the anchor sheath 630, using a push rod (not shown) or other translation mechanism. Thus, the anchor sheath 630 provides an anchor translation channel for deploying anchors to the treatment site.

In FIG. 6A, the delivery sheath 610 and guide sheath 620 are shown in cross section, exposing the anchor sheath 630 for view. In one embodiment, the anchor sheath may comprise a slit 640 extending from a proximal end 602 to a distal end 605 of the anchor sheath 620. In some embodiments, the slit 640 may extend to a proximal handle of the delivery catheter, for example to align with the slot 231 of the anchor feed cylinder 230 (FIG. 2B) of a delivery catheter handle. According to one aspect, the slit 640 exposes the anchor translation channel provided by the anchor sheath 630 to a suture translation channel that is maintained separately from the anchor translation channel (for example, between the delivery sheath 610 and the guide sheath 620) to minimize interaction between suture and anchor repair components.

For example, FIG. 6B is a second view of the catheter 600, where the delivery sheath 610 is shown in cross section to expose the guide sheath 620. The anchor sheath 630 is shown extending from the distal end of the guide sheath 620.

According to one aspect, the guide sheath 620 may have one or more openings extending therethrough, such as window 622. The window may expose a portion of the anchor sheath comprising the slit 640, providing a passageway for the suture to couple with a coupler 624 of an anchor 623, while allowing the guide sheath 620 to keep the suture 650 from tangling with anchors 623.

For example, in one embodiment the slit 640 in the access sheath extends longitudinally through the proximal end of the handle 225 (FIG. 2A) of a delivery catheter. An anchor may be threaded over or tied to suture 650 and pushed through the anchor sheath 630 by advancement of the guide sheath 620 over the anchor sheath. In FIG. 6B, anchor 623 is shown positioned within the anchor sheath 630 such that a coupler 624 of the anchor 623 is oriented towards the slit 640, which in turn is oriented towards the window 622. Translation of the guide sheath 620 over the anchor sheath 630 essentially pulls the anchor 623 longitudinally through the anchor sheath 630 to the treatment site. At the distal end 605 of the catheter 600, the anchor may be released by rotating the guide sheath to release the joined suture 650/ coupler 624 pair. The anchor may then be deployed to target tissue using a push rod (not shown) to push the anchor out of the anchor sheath.

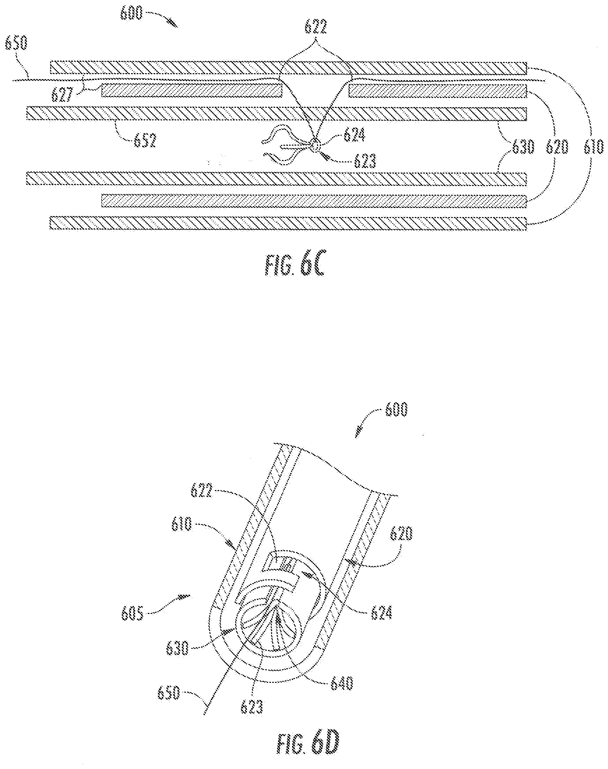

FIG. 6C illustrates a longitudinal cross section of catheter 600, illustrating the anchor sheath 630 disposed within the guide sheath 620 which in turn is disposed within the delivery catheter 610. In FIG. 6C, a sidewall 652 of slit 640 (FIG. 6B) is shown. Anchor 623 is disposed within the anchor sheath 630, and the suture 650 is shown to extend through the window 622 within guide sheath 620, past the sidewall 652 of the slit 640, through anchor coupler 624 of anchor 623, past sidewall 652 and out window 622 to the channel 627 between delivery sheath 610 and guide sheath 620. By providing a system with separate translation channels for the suture 650 and the anchor 623, the potential for entanglement is reduced. The slit 640/window 622 arrangement of the anchor catheter 630/ guide catheter 620 pair further assists with maintaining the orientation of the anchor 623 within the anchor catheter 630.

Although an embodiment having one window 622 configured to deploy one anchor is shown, it is appreciated that other arrangements, for example having guide catheters with two or more windows spaced apart along the longitudinal extend of the guide catheter are within the scope of this disclosure. In such embodiments, the guide catheter/anchor pair arrangement may further assist with controlling a relative spacing of the anchors during deployment.

FIG. 6D illustrates a view of the distal end 605 of the catheter 600 wherein an anchor 623 has been advanced to the distal end of the anchor catheter 630 and is ready for release to a treatment site within a heart cavity. In one embodiment, distal advancement of the anchor 623 to the treatment site is enabled by translating the guide catheter 620 within the delivery catheter 610. As the window 622 advances along the anchor sheath 630 due to advancement of the guide catheter 620 within the delivery catheter 610, the window 622 distally translates the suture 650 within the slit 640, maintaining the position of the anchor coupler 624 relative to window 622.

When the anchor 623 reaches the distal end 605 of the delivery catheter 610, the guide sheath 620 may be advanced past the distal end of the anchor catheter and rotate to release the anchor to the treatment site. In some embodiments, the window 622 may be part of a notched opening at the distal end of the guide catheter as shown in FIG. 6D. With such an arrangement, rotation of the guide catheter may facilitate the release of the suture from the distal end of the anchor catheter.

Accordingly, a delivery catheter and system has been shown and described that uses cooperating sheaths to provide separate translation channels for repair components such as sutures and anchors.

In some embodiments, a delivery catheter may be formed with internal features configured to maintain the orientation of the anchor within the catheter. The internal features may include channels that may be cooperate with features of the anchors or other components to streamline component delivery.

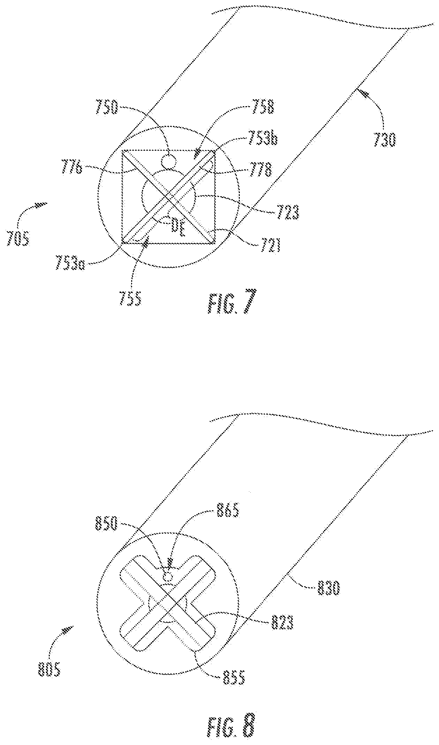

For example, FIG. 7 illustrates one embodiment of a distal end 705 of an anchor catheter 730 including a bore 755 extending therethrough. The bore 755 includes internal features configured to maintain orientation of an anchor 723. For example, the bore 755 may be configured to accept the anchor in a single, predetermined orientation and/or position, and to maintain the predetermined orientation and/or position along the extent of the delivery catheter. Designing the bore of the delivery catheter in such a manner as to preclude rotation of the anchor within the bore reduces the potential for repair component entanglement.

In addition, configuring the bore 755 to limit the movement of the anchor within the anchor catheter in this manner, features of the anchor may be used to provide a suture translation channel that is separated from talons 721 of the anchor 723.

For example, in FIG. 7, the bore 755 is generally rectangular in shape, with diagonal extend D_{E} matched to a maximum distance between anchor talon endpoints 753a, 753b of the anchor 723 when the anchor 723 is in a compressed state within the anchor catheter 730. As such, the anchor 723 is precluded from rotating within the bore 755.

Because the anchor 723 is precluded from rotating within the bore 755, spaces between anchor features may be used as channels to support repair elements without risk of entanglement. For example, a channel 758, defined between anchor talon 776 and 778 may be used to carry the suture 750, for example as the anchor 723 is translated to the treatment site. In embodiments such as that shown in FIG. 7, the anchor may be distally translated using a push rod or other device that is slidably translatable within the bore 755 of the anchor sheath 730.

The anchor sheath 730 may comprise a delivery catheter, or may comprise a separate working catheter, translatable within a delivery catheter for delivering components to the treatment site.

FIG. 8 illustrates an embodiment of a distal end 805 of an anchor catheter 830 having a shaped bore 855, wherein the shape of the bore 855 is generally matched to the size and shape of an anchor 823, to preclude rotation or disorientation of the anchor 823 as it is translated through the anchor sheath 830. In FIG. 8, a channel 865 may be reserved within the bore 855 to support sutures 850. Because the bore 855 is matched in general size and shape to the anchor 823, the likelihood of interference between sutures 850 and the anchor is minimized.

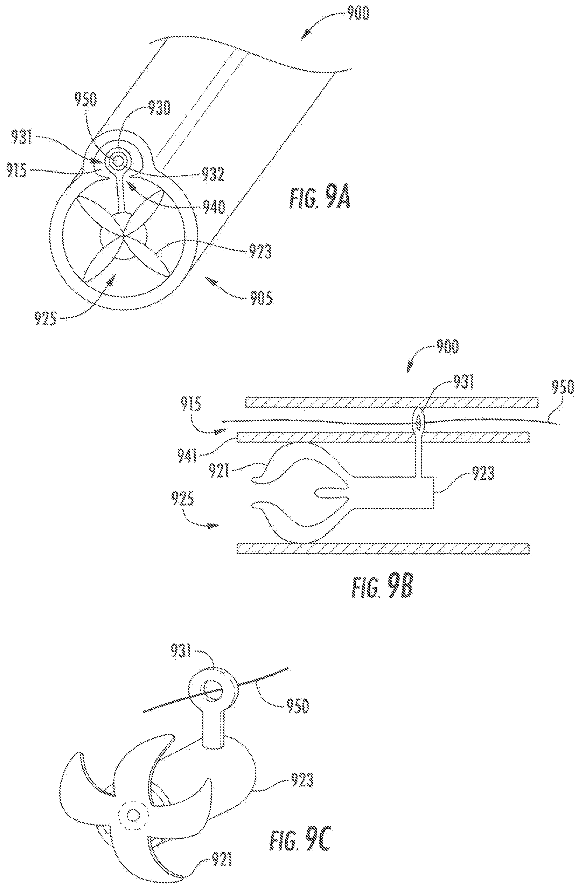

FIGS. 9A - 9C illustrate perspective views of another embodiment of a delivery catheter system for use in deploying repair mechanisms to a treatment site within a heart. It is appreciated that modifications to the anchors as well as the delivery catheter may further assist in maintaining the orientation, spacing and/or separation between repair components such as anchors and sutures.

FIG. 9A illustrates a distal end 905 of a delivery catheter 900 configured to support an anchor, such as anchor 923. Anchor 923 is shown in a pre-deployed state within the delivery catheter 900 in FIGS. 9A and 9B, and in a post-deployed state in FIG. 9C. In FIG. 9A, delivery catheter 900 is shown to include two channels, a suture translation channel 915 and an anchor translation channel 925. A slit 940 couples the suture translation channel 915 to the anchor translation channel 925. During use, in the embodiment of FIG. 9A, the suture 950 may be advanced through the suture translation channel 915. An anchor coupler 930 extends from an anchor 923 that is translatably disposed within the anchor translation channel 925 into the suture translation channel 915 through a slit 940 that joins the two channels. In one embodiment, the anchor coupler 930 includes an eyelet 931 or other opening that provides a suture lumen 932 configured to support a suture 950 along which the anchor 923 may be advanced towards the treatment site.

FIG. 9B is a longitudinal cross section view of the delivery catheter 900. In FIG. 9B, the anchor 923 is disposed such that the talons 921 are in a compressed state to facilitate translation of the anchor 923 through the anchor translation channel 925. Sidewall 941 comprises a sidewall of the slit 940 of FIG. 9A. As shown in FIG. 9B, the eyelet 931 extends up past the sidewall 941 of the slit into the suture translation channel 915. Suture 950 may be attached or threaded through the eyelet 931 as the anchor 923 is introduced into anchor translation channel 925. With such an arrangement, the likelihood of interference between the talons 921 of the anchor 923 and the suture 950 is greatly reduced.

FIG. 9C illustrates an embodiment of the anchor 923, following release from the distal end 905 of the delivery catheter 900 (FIG. 9A). In one embodiment, as the anchor 923 is pushed out of the anchor translation channel 925, the talons 921 of the anchor 923 may return to their native configuration, enabling the talons 921 to expand to engage tissue. As anchors are deployed from the delivery catheter, the talons 921, burrs, barbs or other anchor features may be pushed into tissue, for example by pressure exerted upon the anchor 923 by the distal end of the delivery catheter or other tool, such as a push rod, that may be extended through the anchor translation channel. The suture 950 extends through (or alternatively may be attached to) the eyelet 931 and may be used to bind anchor 923 to previously or subsequently deployed anchors. Such an arrangement facilitates heart repair techniques that deploy and cinch multiple anchors by reducing the opportunity for entanglement and thereby improving technique outcomes.

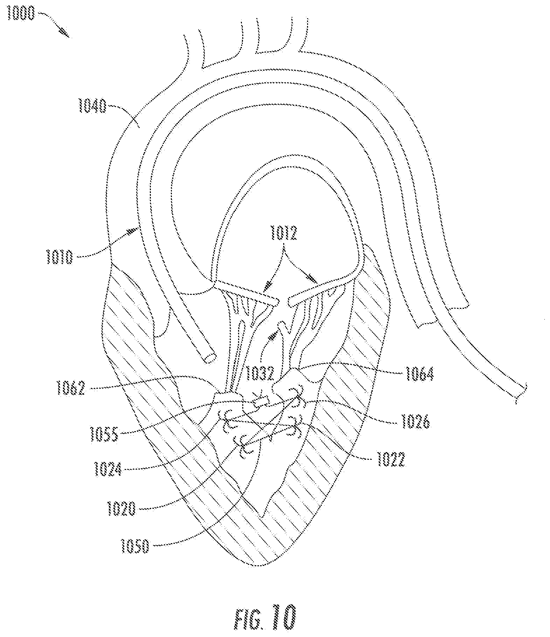

FIG. 10 is a diagram of a heart 1000 that is used herein to describe an exemplary repair technique that may be used to restore mitral valve function when mitral valve function is impaired by degraded function of the chordae tendineae 1032. For example, stretching or rupture of the chordae tendineae 1032 may result in leaflet flailing, wherein the leaflets of the mitral valve 1012 fail to coapt during systole, causing mitral regurgitation.

According to one aspect it is realized that drawing together the anterior papillary muscle 1062 and the posterior papillary muscle 1064 may draw together the valves 1012 and/or reduce the volume of the left ventricle to improve cardiac function.

A delivery catheter such as that described herein may advantageously be used to deploy multiple anchors, such as anchors 1020, 1022, 1024 and 1026 to the papillary treatment site over a single suture 1050. The ends of the suture 1050 may then be cinched to draw together the anchors 1020-1026 and the associated papillary muscles 1062, 1064. A resistive weld, band, or other cinch device 1055 may then be used to join the ends of the suture 1050, and the delivery catheter 1010 may be withdrawn back through the aorta 1040 and removed from the patient. Other methods of deployment, including transapical, transseptal or other approaches may alternatively be used.

While it is appreciated that it may be beneficial in such systems to include a suture 1050 that loops proximally back to the handle to assist with cinching, the present disclosure is not limited to a looped suture. Rather, repair techniques that use a single, non-looped suture, such as that described with regard to FIGS. 5A-5C may benefit from the delivery catheter design principles disclosed herein, that maintain orientation, spacing and/or separation between anchors.

Various modifications to the implementations described in this disclosure will be readily apparent to those skilled in the art, and the generic principles defined herein can be applied to other implementations without departing from the scope of this disclosure. Thus, the disclosure is not intended to be limited to the implementations shown herein but is to be accorded the widest scope consistent with the claims, the principles and the novel features disclosed herein. The word "example" is used exclusively herein to mean "serving as an example, instance, or illustration." Any implementation described herein as an "example" is not necessarily to be construed as preferred or advantageous over other implementations, unless otherwise stated.

Certain features that are described in this specification in the context of separate implementations also can be implemented in combination in a single implementation. Conversely, various features that are described in the context of a single implementation also can be implemented in multiple implementations separately or in any suitable sub-combination. Moreover, although features can be described above as acting in certain combinations and even initially claimed as such, one or more features from a claimed combination can in some cases be excised from the combination, and the claimed combination can be directed to a sub-combination or variation of a sub-combination. Similarly, while operations are depicted in the drawings in a particular order, this should not be understood as requiring that such operations be performed in the particular order shown or in sequential order, or that all illustrated operations be performed, to achieve desirable results. Additionally, other implementations are within the scope of the following claims. **In** some cases, the actions recited in the claims can be performed in a different order and still achieve desirable results.

It will be understood by those within the art that, in general, terms used herein are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

The devices and/or methods disclosed and claimed herein can be made and executed without undue experimentation in light of the present disclosure. While various embodiments of the devices and methods of this disclosure have been described, it may be apparent to those of skill in the art that variations can be applied to the devices and/or methods and in the steps or in the sequence of steps of the method described herein without departing from the concept and scope of the disclosure. All such similar substitutes and modifications apparent to those skilled in the art are deemed to be within the scope of the disclosure as defined by the appended claims

## Claims

1. A delivery catheter (200, 600, 900, 1000) for cardiac repair components (125) comprising:
a first channel extending from a proximal end to a distal end of the delivery catheter, the first channel configured for axial translation of a first component to a treatment site; and
a second channel, extending from the proximal end of the delivery catheter to the distal end of the delivery catheter, the second channel configured for axial translation of a second component to the treatment site;
wherein the first component is coupled to the second component, and the first component is configured for axial translation through the first channel in coordination with axial translation of the second component through the second channel for concurrent deployment of the first component and second component from the delivery catheter;
an axially translatable opening between the first channel and the second channel,
**characterized by**
a guide catheter (620) disposed within the delivery catheter (610);
the first channel disposed between the guide catheter (620) and the delivery catheter (610); and
an anchor catheter (630, 730, 830) disposed within the guide catheter (620), the anchor catheter (630, 730, 830) comprising a slit (640) extending through the anchor catheter (630, 730, 830) from a distal end (605, 705, 805) of the anchor catheter to a proximal end (602) of the anchor catheter, the anchor catheter providing the second channel;
wherein the guide catheter (620) comprises a window (622) extending therethrough, the window (622) slidably disposed over the slit (640) of the anchor catheter (630, 730, 830), wherein the window (622) of the guide catheter (620) and slit (640) of the anchor catheter (630, 730, 830) cooperate to provide the axially translatable opening between the first channel and the second channel.

2. The delivery catheter of claim 1, wherein the first channel and second channel are configured to couple the first component to the second component while limiting interaction between the first component and the second component.

3. The delivery catheter of claim 2, wherein the second channel is configured to limit rotational movement of the second component during translation of the second component through the second channel, and wherein the first channel is disposed within a portion of the second channel defined by the second component.

4. The delivery catheter of claim 3, wherein the second channel comprises a bore (755, 855) sized to limit rotation of the second component, and wherein the first channel is defined by features of the second component.

5. The delivery catheter of claim 4, wherein the bore corresponds in size and shape to the second component.

6. The delivery catheter of claim 2, wherein the second component comprises a body and a coupler for engaging the first component, the first channel is configured to carry the coupler and the second channel is configured to carry the body.

7. A cardiac repair system comprising
the delivery catheter of any of the preceding claims;
a first component comprising a suture (650); and
a second component comprising an anchor (422, 422, 532, 534, 623, 723, 823, 923) having a suture coupling (624),
wherein the suture (650) extends through the suture coupling (624) of the anchor (422, 422, 532, 534, 623, 723, 823, 923).

8. The cardiac repair system of claim 7, wherein the suture coupling (624) is disposed within the second channel.

9. The cardiac repair system of claim 7, wherein the suture coupling (624) is disposed within the first channel.

10. The cardiac repair system of claim 7, further comprising a plurality of anchors (422, 422, 532, 534, 623, 723, 823, 923), wherein the suture (650) extends from a proximal handle (225) of the delivery catheter (600) through the plurality of anchors (422, 422, 532, 534, 623, 723, 823, 923) and back to the proximal handle (225).

11. The cardiac repair system of claim 7, further comprising a plurality of anchors (422, 422, 532, 534, 623, 723, 823, 923), wherein the suture (650) is fixedly attached to a first anchor (422, 422, 532, 534, 623, 723, 823, 923) of the plurality of anchors (422, 422, 532, 534, 623, 723, 823, 923) and extends through each of plurality of anchors (422, 422, 532, 534, 623, 723, 823, 923) to the proximal end of the delivery catheter (600).

12. The cardiac repair system of claim 7, wherein the anchor (422, 422, 532, 534, 623, 723, 823, 923) comprises a first configuration for translation through the delivery catheter and a second configuration for deployment to the treatment site.

## Patentansprüche

1. Zuführkatheter (200, 600, 900, 1000) für Herzreparaturkomponenten (125), umfassend:
einen ersten Kanal, der sich von einem proximalen Ende zu einem distalen Ende des Zuführkatheters erstreckt, wobei der erste Kanal für eine axiale Translation einer ersten Komponente zu einer Behandlungsstelle ausgestaltet ist, und einen zweiten Kanal, der sich von dem proximalen Ende des Zuführkatheters zu dem distalen Ende des Zuführkatheters erstreckt, wobei der zweite Kanal für eine axiale Translation einer zweiten Komponente zu der Behandlungsstelle ausgestaltet ist,
wobei die erste Komponente an die zweite Komponente gekoppelt ist und die erste Komponente zur axialen Translation durch den ersten Kanal in Koordination mit einer axialen Translation der zweiten Komponente durch den zweiten Kanal zum gleichzeitigen Ausbringen der ersten Komponente und der zweiten Komponente aus dem Zuführkatheter ausgestaltet ist,
eine axial translatierbare Öffnung zwischen dem ersten Kanal und dem zweiten Kanal,
**gekennzeichnet durch**
einen Führungskatheter (620), der in dem Zuführkatheter (610) angeordnet ist,
wobei der erste Kanal zwischen dem Führungskatheter (620) und dem Zuführkatheter (610) angeordnet ist, und
einen Ankerkatheter (630, 730, 830), der in dem Führungskatheter (620) angeordnet ist,
wobei der Ankerkatheter (630, 730, 830) einen Schlitz (640) umfasst, der sich durch den Ankerkatheter (630, 730, 830) von einem distalen Ende (605, 705, 805) des Ankerkatheters zu einem proximalen Ende (602) des Ankerkatheters erstreckt, wobei der Ankerkatheter den zweiten Kanal bereitstellt,
wobei der Führungskatheter (620) ein sich dort hindurcherstreckendes Fenster (622) umfasst,
wobei das Fenster (622) verschiebbar über dem Schlitz (640) des Ankerkatheters (630, 730, 830) angeordnet ist, wobei das Fenster (622) des Führungskatheters (620) und der Schlitz (640) des Ankerkatheters (630, 730, 830) zusammenwirken, um die axial translatierbare Öffnung zwischen dem ersten Kanal und dem zweiten Kanal bereitzustellen.

2. Zuführkatheter nach Anspruch 1, wobei der erste Kanal und der zweite Kanal dazu ausgestaltet sind, die erste Komponente an die zweite Komponente zu koppeln, während das Zusammenwirken zwischen der ersten Komponente und der zweiten Komponente begrenzt wird.

3. Zuführkatheter nach Anspruch 2, wobei der zweite Kanal zur Begrenzung der Drehbewegung der zweiten Komponente während der Translation der zweiten Komponente durch den zweiten Kanal ausgestaltet ist und wobei der erste Kanal in einem Abschnitt des zweiten Kanals angeordnet ist, der durch die zweite Komponente definiert ist.

4. Zuführkatheter nach Anspruch 3, wobei der zweite Kanal eine Bohrung (755, 855) umfasst, die so bemessen ist, dass sie die Drehung der zweiten Komponente begrenzt, und wobei der erste Kanal durch Merkmale der zweiten Komponente definiert ist.

5. Zuführkatheter nach Anspruch 4, wobei die Bohrung in Größe und Form der zweiten Komponente entspricht.

6. Zuführkatheter nach Anspruch 2, wobei die zweite Komponente einen Körper und einen Koppler zur Ineingriffnahme der ersten Komponente umfasst, der erste Kanal zum Tragen des Kopplers ausgestaltet ist und der zweite Kanal zum Tragen des Körpers ausgestaltet ist.

7. Herzreparatursystem, umfassend
den Zuführkatheter nach einem der vorhergehenden Ansprüche,
eine erste Komponente, die ein Nahtmaterial (650) umfasst, und
eine zweite Komponente, die einen Anker (422, 422, 532, 534, 623, 723, 823, 923) mit einer Nahtmaterialkoppelung (624) umfasst,
wobei sich das Nahtmaterial (650) durch die Nahtmaterialkoppelung (624) des Ankers (422, 422, 532, 534, 623, 723, 823, 923) erstreckt.

8. Herzreparatursystem nach Anspruch 7, wobei die Nahtmaterialkoppelung (624) in dem zweiten Kanal angeordnet ist.

9. Herzreparatursystem nach Anspruch 7, wobei die Nahtmaterialkupplung (624) in dem ersten Kanal angeordnet ist.

10. Herzreparatursystem nach Anspruch 7, ferner umfassend eine Vielzahl von Ankern (422, 422, 532, 534, 623, 723, 823, 923), wobei sich das Nahtmaterial (650) von einem proximalen Griff (225) des Zuführkatheters (600) durch die Vielzahl von Ankern (422, 422, 532, 534, 623, 723, 823, 923) und zurück zu dem proximalen Griff (225) erstreckt.

11. Herzreparatursystem nach Anspruch 7, ferner umfassend eine Vielzahl von Ankern (422, 422, 532, 534, 623, 723, 823, 923), wobei das Nahtmaterial (650) fest an einem ersten Anker (422, 422, 532, 534, 623, 723, 823, 923) der Vielzahl von Ankern (422, 422, 532, 534, 623, 723, 823, 923) angebracht ist und sich durch jeden der Vielzahl von Ankern (422, 422, 532, 534, 623, 723, 823, 923) zu dem proximalen Ende des Zuführkatheters (600) erstreckt.

12. Herzreparatursystem nach Anspruch 7, wobei der Anker (422, 422, 532, 534, 623, 723, 823, 923) eine erste Konfiguration zur Translation durch den Zuführkatheter und eine zweite Konfiguration zum Ausbringen an der Behandlungsstelle umfasst.

## Revendications

1. Cathéter de pose (200, 600, 900, 1000) pour des composants de réparation cardiaque (125) comprenant :
un premier canal s'étendant d'une extrémité proximale à une extrémité distale du cathéter de pose, le premier canal étant conçu pour une translation axiale d'un premier composant jusqu'à un site de traitement ; et un second canal, s'étendant de l'extrémité proximale du cathéter de pose à l'extrémité distale du cathéter de pose, le second canal étant conçu pour une translation axiale d'un second composant jusqu'au site de traitement ;
le premier composant étant accouplé au second composant, et le premier composant étant conçu pour une translation axiale à travers le premier canal en coordination avec une translation axiale du second composant à travers le second canal pour une mise en place simultanée du premier composant et du second composant à partir du cathéter de pose ;
une ouverture propre à être déplacée axialement par translation entre le premier canal et le second canal, **caractérisé par**
un cathéter de guidage (620) disposé à l'intérieur du cathéter de pose (610) ;
le premier canal étant disposé entre le cathéter de guidage (620) et le cathéter de pose (610) ; et
un cathéter de fixation (630, 730, 830) disposé à l'intérieur du cathéter de guidage (620), le cathéter de fixation (630, 730, 830) comprenant une fente (640) s'étendant à travers le cathéter de fixation (630, 730, 830) d'une extrémité distale (605, 705, 805) du cathéter de fixation à une extrémité proximale (602) du cathéter de fixation, le cathéter de fixation définissant le second canal ;
le cathéter de guidage (620) comprenant une fenêtre (622) s'étendant à travers lui, la fenêtre (622) étant disposée de manière coulissante au-dessus de la fente (640) du cathéter de fixation (630, 730, 830), la fenêtre (622) du cathéter de guidage (620) et la fente (640) du cathéter de fixation (630, 730, 830) coopérant pour définir l'ouverture propre à être déplacée axialement par translation entre le premier canal et le second canal.

2. Cathéter de pose selon la revendication 1, dans lequel le premier canal et le second canal sont conçus pour accoupler le premier composant au second composant tout en limitant une interaction entre le premier composant et le second composant.

3. Cathéter de pose selon la revendication 2, dans lequel le second canal est conçu pour limiter un mouvement de rotation du second composant lors d'une translation du second composant à travers le second canal, et dans lequel le premier canal est disposé à l'intérieur d'une partie du second canal définie par le second composant.

4. Cathéter de pose selon la revendication 3, dans lequel le second canal comprend un orifice (755, 855) dimensionné pour limiter une rotation du second composant, et dans lequel le premier canal est défini par des éléments du second composant.

5. Cathéter de pose selon la revendication 4, dans lequel l'orifice correspond, du point de vue de la taille et de la forme, au second composant.

6. Cathéter de pose selon la revendication 2, dans lequel le second composant comprend un corps et un moyen d'accouplement destiné à entrer en prise avec le premier composant, le premier canal est conçu pour transporter le moyen d'accouplement et le second canal est conçu pour transporter le corps.

7. Système de réparation cardiaque comprenant
le cathéter de pose selon l'une quelconque des revendications précédentes ;
un premier composant comprenant un élément de suture (650) ; et
un second composant comprenant un élément de fixation (422, 422, 532, 534, 623, 723, 823, 923) comportant un moyen d'accouplement d'élément de suture (624),
dans lequel l'élément de suture (650) s'étend à travers le moyen d'accouplement d'élément de suture (624) de l'élément de fixation (422, 422, 532, 534, 623, 723, 823, 923).

8. Système de réparation cardiaque selon la revendication 7, dans lequel le moyen d'accouplement d'élément de suture (624) est disposé à l'intérieur du second canal.

9. Système de réparation cardiaque selon la revendication 7, dans lequel le moyen d'accouplement d'élément de suture (624) est disposé à l'intérieur du premier canal.

10. Système de réparation cardiaque selon la revendication 7, comprenant, en outre, une pluralité d'éléments de fixation (422, 422, 532, 534, 623, 723, 823, 923), dans lequel l'élément de suture (650) s'étend à partir d'une poignée proximale (225) du cathéter de pose (600) à travers la pluralité d'éléments de fixation (422, 422, 532, 534, 623, 723, 823, 923) puis revient à la poignée proximale (225).

11. Système de réparation cardiaque selon la revendication 7, comprenant, en outre, une pluralité d'éléments de fixation (422, 422, 532, 534, 623, 723, 823, 923), dans lequel l'élément de suture (650) est attaché fixement à un premier élément de fixation (422, 422, 532, 534, 623, 723, 823, 923) de la pluralité d'éléments de fixation (422, 422, 532, 534, 623, 723, 823, 923) et s'étend à travers chaque élément de la pluralité d'éléments de fixation (422, 422, 532, 534, 623, 723, 823, 923) jusqu'à l'extrémité proximale du cathéter de pose (600).

12. Système de réparation cardiaque selon la revendication 7, dans lequel l'élément de fixation (422, 422, 532, 534, 623, 723, 823, 923) a une première configuration pour sa translation à travers le cathéter de pose et une seconde configuration pour sa mise en place au niveau du site de traitement.
